# EUROPEAN PATENT SPECIFICATION

(11) **EP 1 611 916 B1**
(45) Date of publication and mention of the grant of the patent: **22.04.2009**
(21) Application number: 04253967.6
(22) Date of filing: 01.07.2004
(51) Int. Cl.: A61M 25/06

(54) **Intravenous catheter introducing device**
Intravenöse Kathetereinführvorrichtung
Dispositif d'introduction intraveineuse d'un cathéter

(43) Date of publication of application: 04.01.2006
(60) Divisional application: 07018178.9
(73) Proprietor: Shue, Ming-Jeng, Hsi Dist, Taichung City (TW); Huang, Deborah, Taichung City (TW); Shue, Phillip, Hsi District, Taichung City (TW)
(72) Inventor: Shue, Ming-Jeng, Hsi Dist, Taichung City (TW); Huang, Deborah, Taichung City (TW); Shue, Phillip, Hsi District, Taichung City (TW)
(74) Representative: Eddowes, Simon

(56) References cited:
- WO-A-02/41932
- WO-A-97/05912
- US-A- 5 575 773
- US-B1- 6 325 781
- US-B1- 6 547 762

## Description

This invention relates to an intravenous catheter introducing device, more particularly to an intravenous catheter introducing device with a needle cannula which is retractable into a barrel for safe disposal.

Referring to Figs. 1 and 2, a self-retracting IV catheter 7introducer disclosed in U.S. patent No. 5,989,220 is shown to include a catheter connector assembly including a barrel 10 enclosing a retraction body 11 which has a front portion 111 that carries a needle cannula 12 and that extends through an opening in front of the barrel 10, and a rear portion 112 that is mounted within the barrel 10. The front portion 111 has a connection surface 111a which is frictionally engaged with a corresponding connection surface 131 of a catheter hub 13 so as to prevent rearward retraction of the retraction body 11. A biasing spring 15 is compressed against a ledge 101 at the front of the barrel 10, and a spring seat 113 of the retraction body 11. In use, the needle cannula 12 which extends through a flexible catheter 14 is inserted into a patient's vein to introduce the catheter 14 into the vein for intravenous delivery of fluid. The catheter hub 13 is then separated from the retraction body 11 by forcibly pulling the barrel 10 while holding the catheter hub 13 so that the catheter hub 13 is separated from the front portion 111 of the retraction body 11 by loosening of the connection surface 111a, and the retraction body 11 is immediately and automatically forced into the barrel 10 by the biasing spring 15, thereby drawing the used needle cannula 12 behind it. However, since the needle cannula 12 is drawn as soon as the catheter hub 13 is disengaged from the retraction body 11, the drawing operation is not manually controllable, which may lead to an undesirable withdrawal of the needle cannula 12 that may result in an accident, such as dropping of the barrel 10.

Furthermore, WO 02/41932 discloses a catheter insertion device with a needle tip protective system for peripheral blood vessel catheterisation. The device comprises a handle and a needle hub disposed inside the handle and includes a flash chamber. The device is arranged in a manner which does not confer the beneficial properties in accordance with the present invention.

The object of the present invention is to provide an intravenous catheter introducing device which can be operated easily and safely to retract a used needle cannula with one hand.

According to this invention, the intravenous catheter introducing device includes a barrel, a needle cannula, a needle hub, a releasably retaining member, an actuator, and a catheter connection assembly as defined in claim 1.

The barrel has front and rear open ends opposite to each other in a longitudinal direction, and a surrounding barrel wall interconnecting and interposed between the front and rear open ends. The surrounding barrel wall includes a front smaller-diameter wall portion and a rear larger-diameter wall portion which are opposite to each other in the longitudinal direction and which are proximate to the front and rear open ends, respectively. The surrounding barrel wall has an inner barrel wall surface which surrounds an axis in the longitudinal direction and which confines a passage that is communicated with the front and rear open ends, and an outer barrel wall surface opposite to the inner barrel wall surface in radial directions relative to the axis.

The needle cannula has a front segment terminating at a tip end, and a rear connecting end opposite to the front segment along the axis.

The needle hub includes a front holding portion and a rear shell portion disposed opposite to each other along the axis. The rear shell portion is inserted into the passage from the rear open end, and is slidable relative to the surrounding barrel wall along the axis between front and rear positions to be proximate to the front open end and the rear open end, respectively. The front holding portion holds the rear connecting end of the needle cannula such that when the rear shell portion is in the front position, the needle cannula is placed in a position of use, where the front segment extends forwardly of the front open end for ready use, and when the rear shell portion is in the rear position, the needle cannula is placed in a disposal position, where the front segment retreats into the passage. The rear shell portion surrounds the axis and defines a flashback chamber fluidly communicated with the needle cannula.

The releasably retaining member is disposed to arrest axial movement of the needle hub relative to the barrel when the rear shell portion of the needle hub is in the front position, and includes a retaining hole and an engaging peg. The retaining hole is formed in the outer barrel wall surface of the larger-diameter wall portion, and extends in a radial direction through the inner barrel wall surface. The engaging peg is disposed to extend in the radial direction, and is engageable in the retaining hole to establish an interengagement between the larger-diameter wall portion and the rear shell portion such that movement of the rear shell portion of the needle hub at the front position is arrested.

The actuator is operable externally and is disposed to enable the engaging peg to be disengaged from the retaining hole so as to permit the axial movement of the needle hub to the rear position.

The catheter connection assembly includes a catheter hub and a tubular catheter. The catheter hub includes a sleeve portion which is detachably sleeved relative to the front holding portion of the needle hub and which defines a duct along the axis, and a tip portion which is opposite to the sleeve portion along the axis, and which defines a through hole that is communicated with the duct along the axis and that permits extension of the front segment therethrough. The tubular catheter has a proximate segment which is inserted into the through hole and which extends along the axis to be fluidly communicated with the duct, and a distal segment which extends from the proximate segment along the axis to extend forwardly of the tip portion so as to surround and sheathe the front segment of the needle cannula while permitting the tip end to project forwardly of the distal segment when the needle cannula is placed in the position of use.

Other features and advantages of the present invention will become apparent in the following detailed description of the preferred embodiments of the invention, with reference to the accompanying drawings, in which:

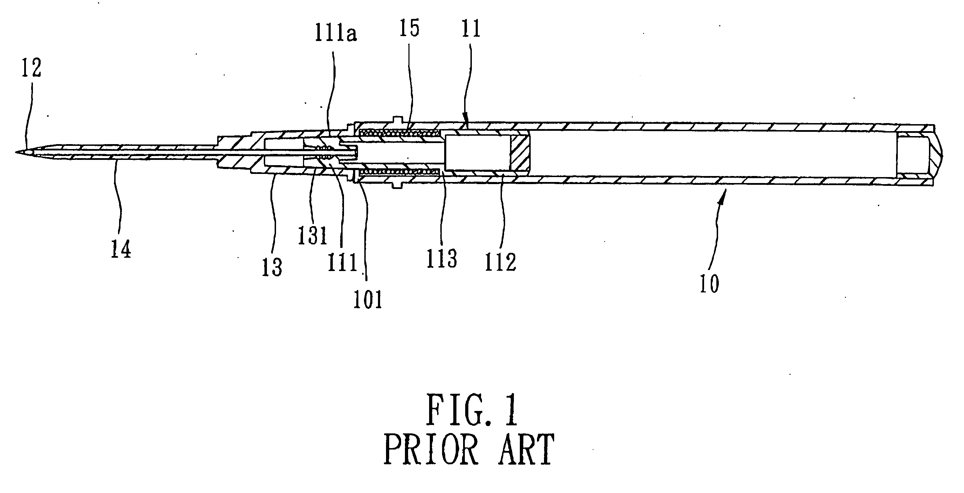

Fig. 1 is a sectional view of a conventional IV catheter introducer in a ready-to-use position;

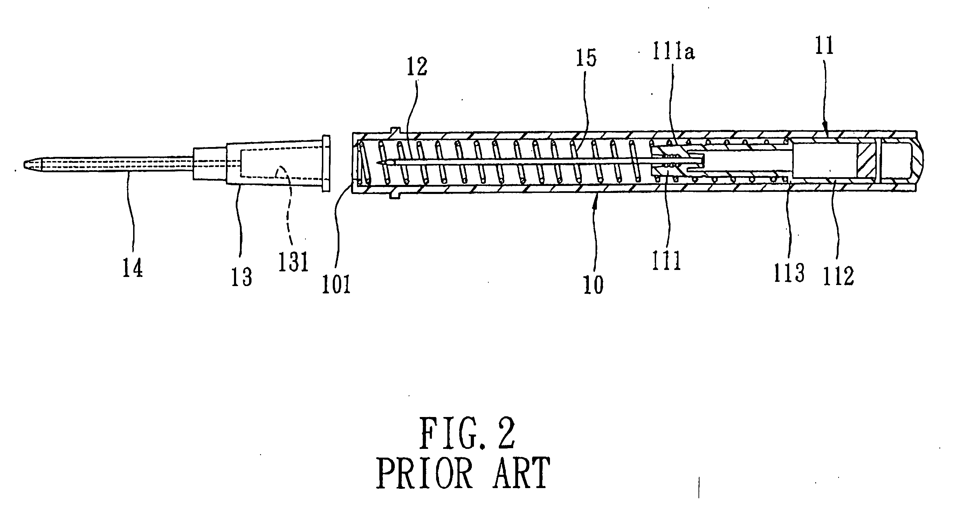

Fig. 2 is a sectional view of the conventional IV catheter introducer in a retracted position;
Fig. 3 is an exploded sectional view of a first preferred embodiment of an intravenous catheter introducing device according to this invention;
Fig. 4 is a sectional view of the first preferred embodiment in a ready-to-use position;
Fig. 5 is a sectional view of the first preferred embodiment in a retracted position;
Fig. 6 is a sectional view of a second preferred embodiment of an intravenous catheter introducing device according to this invention in a ready-to-use position;
Fig. 7 is a sectional view of the second preferred embodiment in a retracted position;
Fig. 8 is a sectional view of a third preferred embodiment of an intravenous catheter introducing device according to this invention in a ready-to-use position;
Fig. 9 is a sectional view of the third preferred embodiment in a retracted position;
Fig. 10 is a sectional view of a fourth preferred embodiment of an intravenous catheter introducing device according to this invention in a ready-to-use position;
Fig. 11 is a sectional view of the fourth preferred embodiment in a retracted position;
Fig. 12 is a fragmentary exploded sectional view of a fifth preferred embodiment of an intravenous catheter introducing device according to this invention; and
Fig. 13 is a sectional view of a sixth preferred embodiment of an intravenous catheter introducing device according to this invention in a ready-to-use position.

Before the present invention is described in greater detail, it should be noted that same reference numerals have been used to denote like elements throughout the specification.

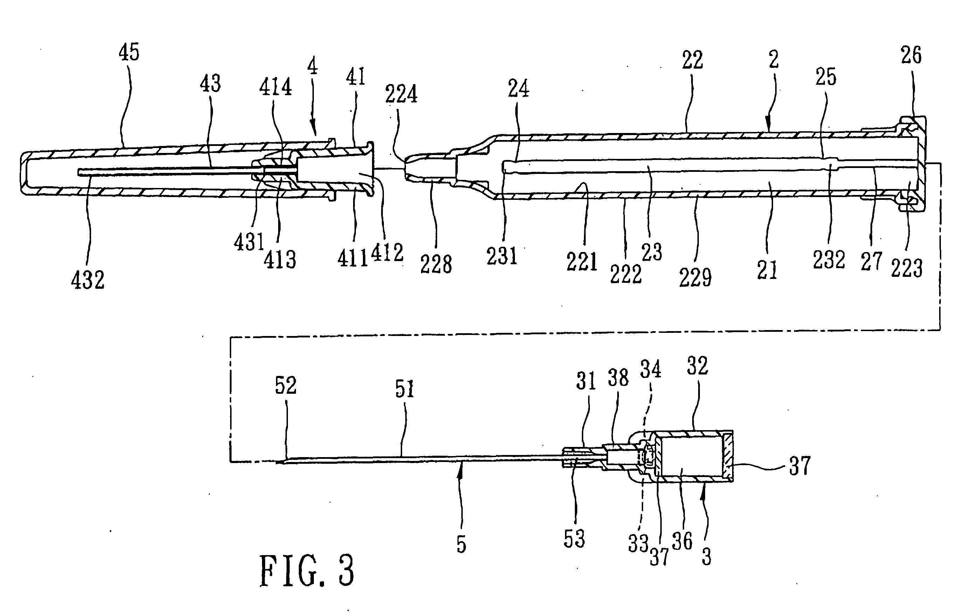

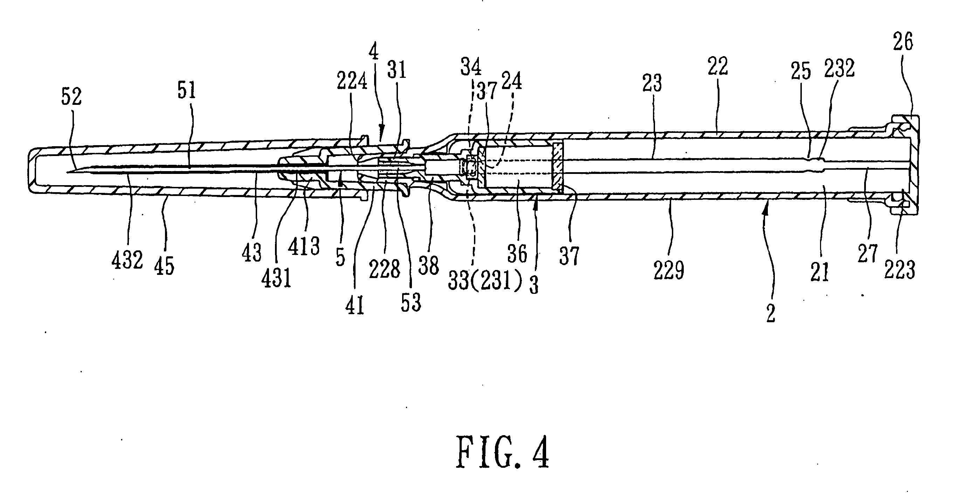

Referring to Figs. 3 and 4, the preferred embodiment of an intravenous catheter introducing device according to the present invention is shown to comprise a barrel 2, a needle hub 3, a needle cannula 5, and a catheter connection assembly 4.

The barrel 2 has front and rear open ends 224,223 opposite to each other in a longitudinal direction, and a surrounding barrel wall 22 which interconnects and which is interposed between the front and rear open ends 224,223. The surrounding barrel wall 22 includes a front smaller-diameter wall portion 228 and a rear larger-diameter wall portion 229 which are opposite to each other in the longitudinal direction and which are proximate to the front and rear open ends 224,223, respectively. The surrounding barrel wall 22 has an inner barrel wall surface 221 which surrounds an axis in the longitudinal direction and which confines a passage 21 communicated with the front and rear open ends 224,223, and an outer barrel wall surface 222 opposite to the inner barrel wall surface 221 in radial directions relative to the axis.

A releasably retaining member includes a retaining hole 231 which is formed in the outer barrel wall surface 222 of the larger-diameter wall portion 229, and which extends in a radial direction through the inner barrel wall surface 221. The rear larger-diameter wall portion 229 has an elongated guideway 23 which extends from the outer barrel wall surface 222 through the inner barrel wall surface 221 in the radial direction, and which is elongated from the retaining hole 231 rearwardly and in the longitudinal direction to terminate at a rear retaining end 232. The elongated guideway 23 has front and rear constricted regions 24,25 which are formed immediately behind the retaining hole 231 and immediately in front of the rear retaining end 232, respectively.

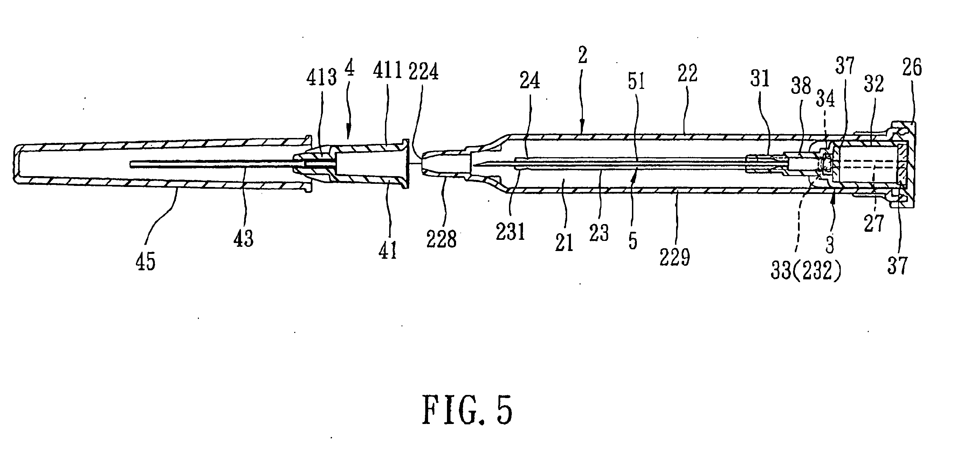

The needle cannula 5 has a front segment 51 terminating at a tip end 52, and a rear connecting end 53 opposite to the front segment 51 along the axis. The needle hub 3 is inserted into the passage 21 from the rear open end 223, which is closed by a closure cap 26, and is slidable relative to the surrounding barrel wall 22 along the axis between front and rear positions to be proximate to the front open end 224 and the rear open end 223, respectively. The needle hub 3 includes a front holding portion 31 which holds the rear connecting end 53 of the needle cannula 5 such that when the needle hub 3 is in the front position, the needle cannula 5 is placed in a position of use, as shown in Fig. 4, where the front segment 51 extends forwardly of the front open end 224 for ready use, and when the needle hub 3 is in the rear position, the needle cannula 5 is placed in a disposal position, as shown in Fig. 5, where the front segment 51 retreats into the passage 21. The needle hub 3 further includes a rear shell portion 32 which is disposed opposite to the front holding portion 31 along the axis and which is received in the passage 21 at the larger-diameter wall portion 229. The rear shell portion 32 surrounds the axis and defines a flashback chamber 36 which is fluidly communicated with the needle cannula 5. Two air-permeable members 37 are in engagement with the rear shell portion 32 to enclose the flashback chamber 36, and are made from a porous filter material for passage of air displaced by the fluid so as to restrain the possible fast flashback blood flow. In addition, the needle hub 3 has an intermediate portion 38 which interconnects the front holding portion 31 and the rear shell portion 32 to communicate the needle cannula 5 with the flashback chamber 36 and which is light transmissible to permit viewing of blood flowing therethrough.

The releasably retaining member further includes an engaging peg 33 disposed on and extending in the radial direction from the rear shell portion 32 to terminate at a shifted end which extends radially and outwardly of the outer barrel wall surface 222. The engaging peg 33 is slidable along the elongated guideway 23 from the retaining hole 231 to the rear retaining end 232 when the needle hub 3 slides from the front position to the rear position. Thus, the engaging peg 33 is engageable in the retaining hole 231 or the rear retaining end 232 to form an interengagement between the larger-diameter wall portion 229 and the rear shell portion 32. When the needle hub 3 is disposed at the front or rear position, axial movement of the needle hub 3 relative to the barrel 2 is arrested by a corresponding one of the front and rear constricted regions 24,25. Once the engaging peg 33 is forced through one of the front and rear constricted regions 24,25, movement of the engaging peg 33 is arrested by virtue of a snap-fit in a corresponding one of the retaining hole 231 and the rear retaining end 232 so as to position the needle hub 3 in a corresponding one of the front and rear positions. The larger-diameter wall portion 229 further has a split 27 which extends from the rear retaining end 232 to the rear open end 223 so as to vest the elongated guideway 23 with an increased flexibility along the radial direction, thereby facilitating the forced movement of the engaging peg 33 through the front and rear constricted regions 24,25, and facilitating the insertion of the engaging peg 33 into the elongate guideway 23 through the split 27.

An enlarged actuator 34 is formed integrally with the shifted end of the engaging peg 33, and is disposed outwardly of and is slidable relative to the outer barrel wall surface 222 so as to be disengaged from the retaining hole 231, thereby permitting the axial movement of the needle hub 3 to the rear position along the elongated guideway 23.

The catheter connection assembly 4 includes a catheter hub 41, a flexible tubular catheter 43, and a tip protector 45.

The catheter hub 41 includes a sleeve portion 411 which is detachably sleeved on the smaller-diameter wall portion 228 of the barrel 2 and which defines a duct 412 along the axis, and a tip portion 413 which is opposite to the sleeve portion 411 along the axis, and which defines a through hole 414 that communicates with the duct 412 along the axis, and that permits extension of the front segment 51 of the needle cannula 5 therethrough.

The tubular catheter 43 has a proximate segment 431 which is inserted into the through hole 414 and which extends along the axis to be fluidly communicated with the duct 412, and a distal segment 432 which extends from the proximate segment 431 along the axis to be disposed forwardly of the tip portion 413 of the catheter hub 41 so as to surround and sheathe the front segment 51 of the needle cannula 5 while permitting the tip end 52 to project forwardly of the distal segment 432 when the needle cannula 5 is placed in the position of use.

In use, after the tip protector 45 is removed, the tip end 52 of the needle cannula 5 is inserted into the patient's vein so as to introduce the tubular catheter 43 into the vein. Blood flowing into the flashback chamber 36 is visible from the intermediate portion 38 of the needle hub 3 so that the user can check whether the needle cannula 5 has been inserted properly into the vein. Referring to Fig. 5, the user can then separate the catheter hub 41 from the barrel 2 by holding the catheter hub 41 with one hand and holding and pulling the surrounding barrel wall 22 with the other hand. At the same time, the actuator 34 is operated with a finger of the hand holding the surrounding barrel wall 22 to move the engaging peg 33 rearwardly along the elongated guideway 23 so as to bring the needle hub 3 to the rear position, thereby placing the needle cannula 5 in the disposal position, where the front segment 51 retreats inwardly and rearwardly of the front open end 224 for safe disposal.

As illustrated, during operation, the user can hold the barrel 2 with one hand and operate the actuator 34 with a finger of the hand to cause the needle hub 3 to move to the rear position for drawing the used needle cannula 5 into the passage 21. Therefore, the operation is controllable by the user and is convenient to conduct. Besides, undesirable accidents can be avoided.

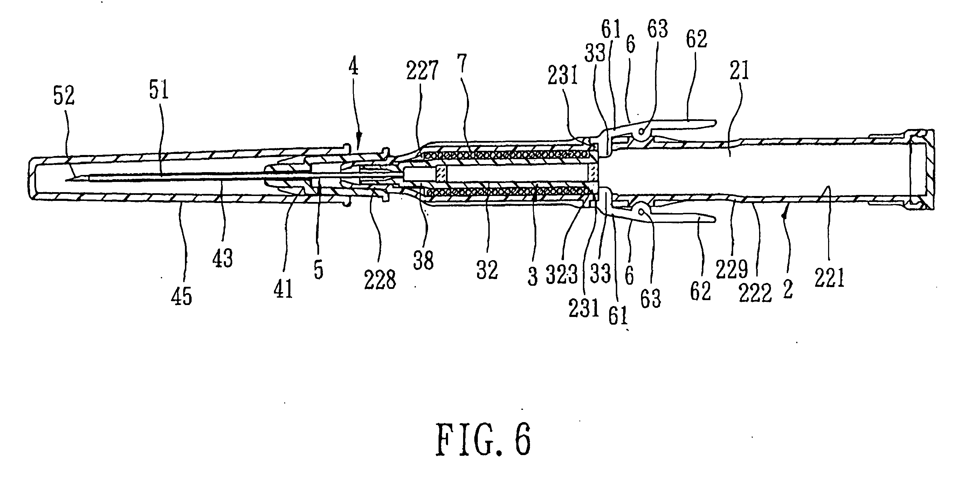

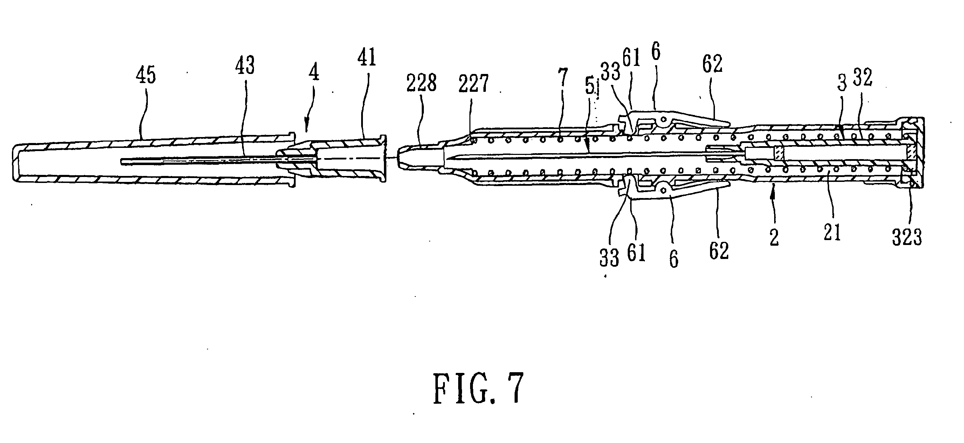

Referring to Figs. 6 and 7, the second preferred embodiment of an intravenous catheter introducing device according to this invention is shown to be similar to the first preferred embodiment in construction. The differences reside in that the releasably retaining member includes two retaining holes 231 formed in the larger-diameter wall portion 229, and that the actuator includes two triggering members 6 formed as levers. Each of the triggering members 6 is mounted pivotally on the outer barrel wall surface 222 at a fulcrum point 63, and includes a weight end 61 formed integrally with the engaging peg 33, and a power end 62 disposed at an opposite side of the weight end 61 relative to the fulcrum point 63 so as to be actuated to move the engaging peg 33 in the radial direction to withdraw the engaging peg 33 from the passage 21 in the barrel 2 to thereby release the needle hub 3.

Furthermore, the inner barrel wall surface 221 of the larger-diameter wall portion 229 has a shoulder 227 with ribs formed adjacent to the smaller-diameter wall portion 228. The rear shell portion 32 of the needle hub 3 has an annular rear flange 323 confronting the shoulder 227 in the longitudinal direction so as to define a biasing member receiving space therebetween and outside of the rear shell portion 32. A biasing member, such as a coiled spring 7, is received in the biasing member receiving space, and includes front and rear spring ends abutting against the ribs of the shoulder 227 and the flange 323, respectively, such that the coiled spring 7 is compressed by the needle hub 3 when the needle hub 3 is in the front position. Due to the provision of the coiled spring 7, when the power ends 62 of the triggering members 6 are depressed at the same time with the fingers to smoothly retract the engaging pegs 33 radially, the needle hub 3 is moved to the rear position so as to bring the needle cannula 5 to the disposal position, thereby preventing shaking of the needle cannula 5 during the retraction of the needle cannula 5 to help lessen the patient's pain.

It is noted that although the actuator in this embodiment is exemplified as including two triggering members 6, one triggering member 6 will be sufficient to perform the function of retaining and releasing the needle hub 3.

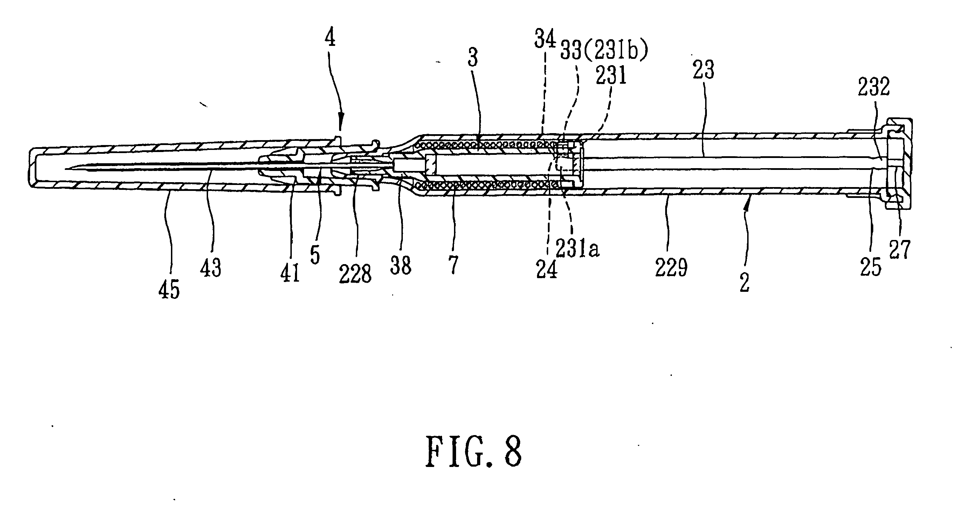

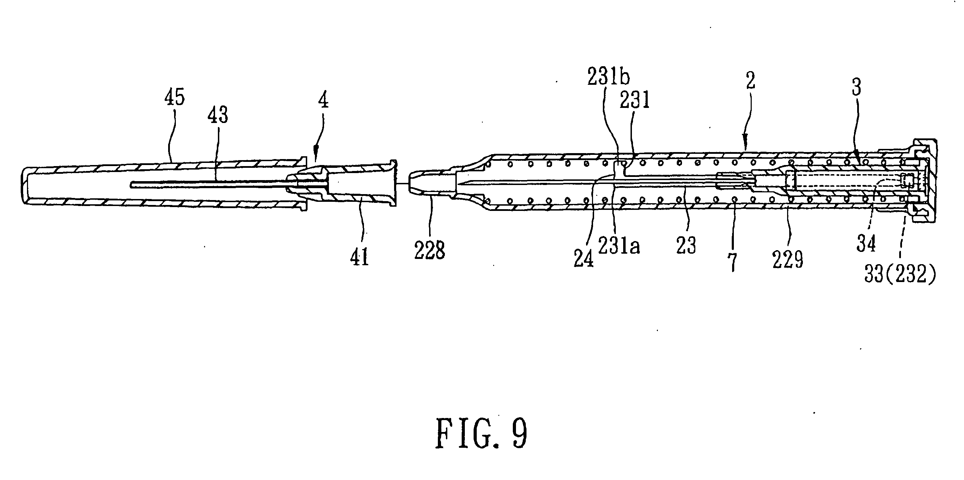

Referring to Figs. 8 and 9, the third preferred embodiment of an intravenous catheter introducing device according to this invention is shown to be similar to the first preferred embodiment in construction. In this embodiment, the retaining hole 231 includes a proximate connecting end 231a and a distal retaining end 231b which are opposite to each other in a transverse direction relative to the longitudinal direction, and which are proximate to and distal from the elongated guideway 23, respectively. The front constricted region 24 is formed between the proximate and distal connecting ends 231a,231b. As such, the engaging peg 33 is engaged in the distal retaining end 231b to arrest the needle hub 3 at the front position. Upon retraction of the needle cannula 5, the actuator 34 is operated to move the engaging peg 33 from the distal retaining end 231b to the proximate connecting end 231a so as to permit rearward movement of the needle hub 3 along the elongated guideway 23. A coiled spring 7 is also provided to bias the needle hub 3 to the rear position as in the second preferred embodiment.

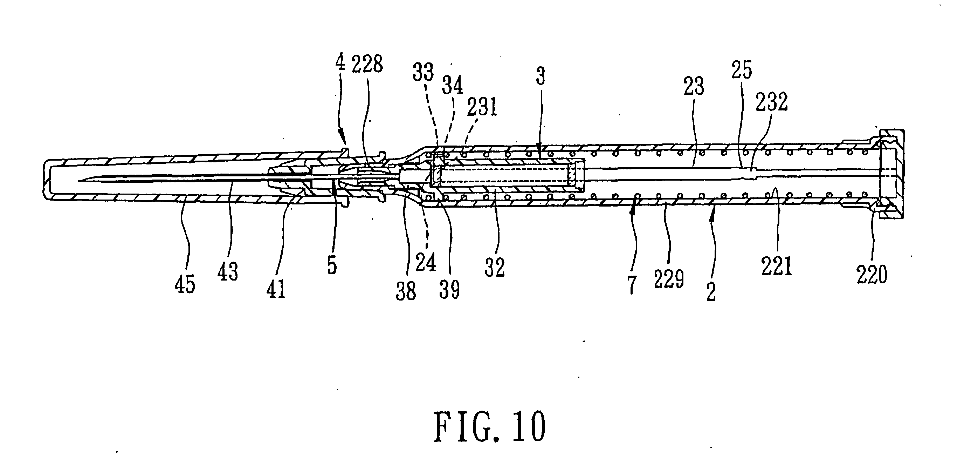

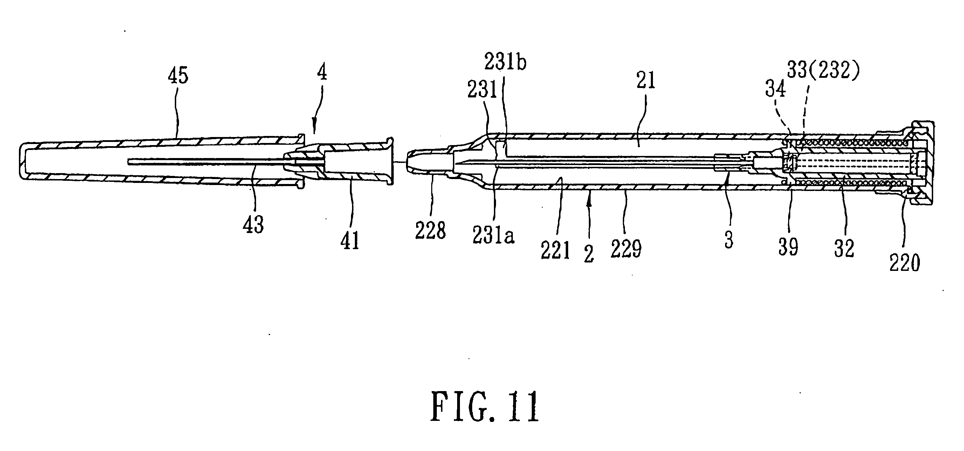

Referring to Figs. 10 and 11, the fourth preferred embodiment of an intravenous catheter introducing device according to this invention is shown to be similar to the third preferred embodiment in construction. The difference resides in that the rear shell portion 32 of the needle hub 3 and the inner barrel wall surface 221 of the larger-diameter wall portion 229 respectively have an annular flange 39 and an annular rear edge 220, which are opposite to and which confront each other in the longitudinal direction so as to define a biasing member receiving space therebetween and outside of the rear shell portion 32. The coiled spring 7 is received in the biasing member receiving space, and has front and rear spring ends secured to the flange 39 and the edge 220, respectively, such that the coiled spring 7 is tensioned by the needle hub 3 when the needle hub 3 is in the front position, as shown in Fig. 10. As illustrated in the third preferred embodiment, referring to Fig 11, by operating the actuator 34 to move the engaging peg 33 from the distal retaining end 231b to the proximate connecting end 231a of the retaining hole 231, rearward movement of the needle hub 3 along the guideway 23 is permitted to enable the needle cannula 5 to be retracted into the passage 21.

## Claims

1. An intravenous catheter introducing device comprising:
a barrel (2) having front and rear open ends (224,223) opposite to each other in a longitudinal direction, and a surrounding barrel wall (22) which interconnects and which is interposed between said front and rear open ends (224,223), said surrounding barrel wall (22) including a front smaller-diameter wall portion (228) and a rear larger-diameter wall portion (229) which are opposite to each other in the longitudinal direction and which are proximate to said front and rear open ends (224,223), respectively, said surrounding barrel wall (22) having an inner barrel wall surface (221) which surrounds an axis in the longitudinal direction and which confines a passage (21) that is communicated with said front and rear open ends (224,223), and an outer barrel wall surface (222) opposite to said inner barrel wall surface (221) in radial directions relative to the axis;
a needle cannula (5) having a front segment (51) terminating at a tip end (52), and a rear connecting end (53) opposite to said front segment (51) along the axis;
a needle hub (3) including a front holding portion (31) and a rear shell portion (32) disposed opposite to each other along the axis, said front holding portion (31) being received in said passage (21), said rear shell portion (32) being inserted into said passage (21) from said rear open end (223), and being slidable relative to said surrounding barrel wall (22) along the axis between front and rear positions to be proximate to said front open end (224) and said rear open end (223), respectively, said front holding portion (31) holding said rear connecting end (53) of said needle cannula (5) such that when said rear shell portion (32) is in the front position, said needle cannula (5) is placed in a position of use, where said front segment (51) extends forwardly of said front open end (224) for ready use, and when said rear shell portion (32) is in the rear position, said needle cannula (5) is placed in a disposal position, where said front segment (51) retreats into said passage (21), said rear shell portion (32) surrounding the axis and defining a flashback chamber (36) which is fluidly communicated with said needle cannula (5),
a releasably retaining member which is disposed to arrest axial movement of said needle hub (3) relative to said barrel (2) when said rear shell portion (32) is in the front position, and which includes
a retaining hole (231) formed in said outer barrel wall surface (222) of said larger-diameter wall portion (229), and extending in a radial direction through said inner barrel wall surface (221), and
an engaging peg (33) disposed to extend in the radial direction, and engageable in said retaining hole (231) to establish an interengagement between said larger-diameter wall portion (229) and said rear shell portion (32) such that movement of said rear shell portion (32) at the front position is arrested;
an actuator (34) operable externally and disposed to enable said engaging peg (33) to be disengaged from said retaining hole (231) so as to permit the axial movement of said needle hub (3) to the rear position;
a catheter hub (41) including a sleeve portion (411) which is detachably sleeved on said smaller-diameter wall portion (228) [relative to said front holding portion (31) of said needle hub (3)] and which defines a duct (412) along the axis, and a tip portion (413) which is opposite to said sleeve portion (411) along the axis, and which defines a through hole (414) that is communicated with said duct (412) along the axis and that permits extension of said front segment (51) therethrough; and
a tubular catheter (43) having a proximate segment (431) which is inserted into said through hole (414) and which extends along the axis to be fluidly communicated with said duct (412), and a distal segment (432) which extends from said proximate segment (431) along the axis to extend forwardly of said tip portion (413) so as to surround and sheathe said front segment (51) of said needle cannula (5) while permitting said tip end (52) to project forwardly of said distal segment (432) when said needle cannula (5) is placed in the position of use, **characterised in that** said front holding portion (31) is surrounded by said smaller-diameter wall portion (228) when in the front position.

2. The intravenous catheter introducing device of Claim 1, **characterized in that** said needle hub (3) further includes an intermediate portion (38) which interconnects said front holding portion (31) and said rear shell portion (32) to communicate said needle cannula (5) with said flashback chamber (36) and which is light transmissible to permit viewing of blood flowing therethrough.

3. The intravenous catheter introducing device of Claim 2, **characterized in that** said needle hub (3) further includes an air-permeable member (37) which is in engagement with said rear shell portion (32) so as to close said flashback chamber (36).

4. The intravenous catheter introducing device of Claim 3, **characterized in that** said air-permeable member (37) is made from a porous filter material.

5. The intravenous catheter introducing device of Claim 1, **characterized in that** said rear larger-diameter wall portion (229) has an elongated guideway (23) extending from said outer barrel wall surface (222) through said inner barrel wall surface (221) in the radial direction, and elongated from said retaining hole (231) rearwardly and in the longitudinal direction to terminate at a rear retaining end (232),
said engaging peg (33) being disposed on and extending radially from said rear shell portion (32) to terminate at a shifted end which extends radially and outwardly of said outer barrel wall surface (222), and being slidable along said elongated guideway (23) from said retaining hole (231) to said rear retaining end (232) when said rear shell portion (32) of said needle hub (3) slides from the front position to the rear position,
said actuator (34) being connected to said shifted end of said engaging peg (33), and being disposed outwardly of and being slidable relative to said outer barrel wall surface (222).

6. The intravenous catheter introducing device of Claim 5, **characterized in that** said elongated guideway (23) has front and rear constricted regions (24,25) which are formed immediately behind said retaining hole (231) and immediately in front of said rear retaining end (232), respectively, such that once said engaging peg (33) is forced through one of said front and rear constricted regions (24,25), movement of said engaging peg (33) is arrested by virtue of a snap-fit in a corresponding one of said retaining hole (231) and said rear retaining end (232) so as to place said needle hub (3) in a corresponding one of the front and rear positions.

7. The intravenous catheter introducing device of Claim 6, **characterized in that** said rear larger-diameter wall portion (229) further has a split (27) which extends from said rear retaining end (232) of said elongated guideway (23) to said rear open end (223).

8. The intravenous catheter introducing device of Claim 5, **characterized in that** said retaining hole (231) includes a proximate connecting end (231a) and a distal retaining end (231b) which are opposite to each other in a transverse direction relative to the longitudinal direction and which are proximate to and distal from said elongated guideway (23), respectively, such that said engaging peg (33) is engaged in said distal retaining end (231b) to arrest movement of said rear shell portion (32) of said needle hub (3) at the front position, and such that said actuator (34) is operated to move said engaging peg (33) from said distal retaining end (231b) to said proximate connecting end (231a) so as to permit slidable movement of said engaging peg (33) along said elongated guideway (23).

9. The intravenous catheter introducing device of Claim 8, further **characterized by** a biasing member (7) which is interposed between said rear shell portion (32) and said inner barrel wall surface (221), and which is disposed to bias said needle hub (3) toward the rear position.

10. The intravenous catheter introducing device of Claim 9, **characterized in that** said inner barrel wall surface (221) of said larger-diameter wall portion (229) and said rear shell portion (32) respectively have an annular shoulder (227) and a flange (323) which are opposite to and which confront each other in the longitudinal direction so as to define a biasing member receiving space therebetween, said biasing member (7) being a coiled spring (7) which has front and rear spring ends abutting against said annular shoulder (227) and said flange (323), respectively, such that said coiled spring (7) is compressed by said needle hub (3) when said rear shell portion (32) is in the front position.

11. The intravenous catheter introducing device of Claim 9, **characterized in that** said rear shell portion (32) and said inner barrel wall surface (221) of said larger-diameter wall portion (229) respectively have an annular flange (39) and an edge (220) which are opposite to and which confront each other in the longitudinal direction so as to define a biasing member receiving space therebetween, said biasing member (7) being a coiled spring (7) which has front and rear spring ends secured to said annular flange (39) and said edge (220), respectively, such that said coiled spring (7) is tensioned by said needle hub (3) when said rear shell portion (32) is in the front position.

12. An intravenous catheter introducing device according to claim 1, **characterised in that** said actuator (34) includes a triggering member (6) which is pivotally mounted on said outer barrel wall surface (222) at a fulcrum point (63), and which includes a weight end (61) that is formed integrally with said engaging peg (33), and that is disposed rearwards from said rear shell portion (32) so as to bring said engaging peg (33) to abut against said rear shell portion (32) when said needle cannula (5) is in the position of use, and a power end (62) disposed at an opposite side of said weight end (61) relative to said fulcrum point (63) so as to be actuated to move said engaging peg (33) in the radial direction to withdraw said engaging peg (33) from said passage (21);
a biasing member (7) which is disposed between said rear shell portion (32) and said inner barrel wall surface (221) to bias said needle hub (3) toward the rear position.

## Patentansprüche

1. Intravenöse Kathetereinführvorrichtung, umfassend:
einen Zylinder (2) mit vorderen und hinteren offenen Enden (224, 223), die sich in einer Längsrichtung gegenüberliegen, und eine umgebende Zylinderwand (22), welche die vorderen und hinteren offenen Enden (224, 223) miteinander verbindet und die dazwischen positioniert ist, wobei die umgebende Zylinderwand (22) ein vorderes Wandteil (228) mit kleinerem Durchmesser und ein hinteres Wandteil (229) mit größerem Durchmesser einschließt, die sich in der Längsrichtung gegenüberliegen und die sich unmittelbar neben den vorderen bzw. hinteren offenen Enden (224, 223) befinden, wobei die umgebende Zylinderwand (22) Folgendes aufweist: eine innere Zylinderwandoberfläche (221), welche eine Achse in der Längsrichtung umgibt und welche eine Passage (21) begrenzt, die sich in Kommunikation mit den vorderen und hinteren offenen Enden (224, 223) befindet, und eine äußere Zylinderwandoberfläche (222), die der inneren Zylinderwandoberfläche (221) in radialer Richtung relativ zur Achse gegenüberliegt;
eine Nadel/Kanüle (5) mit einem Vordersegment (51), das an einem Spitzenende (52) endet, und einem hinteren Verbindungsende (53), das gegenüber dem Vordersegment (51) entlang der Achse liegt;
einen Nadelansatz (3), der Folgendes einschließt: ein vorderes Halteteil (31) und ein hinteres Mantelteil (32), die sich gegenüberliegend entlang der Achse angeordnet sind, wobei das vordere Halteteil (31) in der Passage (21) aufgenommen wird, wobei das hintere Mantelteil (32) in die Passage (21) vom hinteren offenen Ende (223) insertiert wird, und relativ zur umgebenden Zylinderwand (22) entlang der Achse zwischen den vorderen und hinteren Positionen gleitbar ist, um sich unmittelbar neben dem vorderen offenen Ende (224) bzw. hinteren offenen Ende (223) zu befinden, wobei das vordere Halteteil (31) das hintere Verbindungsende (53) der Nadel/Kanüle (5) dergestalt hält, dass wenn sich das hintere Mantelteil (32) in der vorderen Position befindet, die Nadel/Kanüle (5) in eine gebrauchsbereite Position gebracht wird, wobei das Vordersegment (51) vom vorderen offenen Ende (224) gebrauchsbereit nach vom verläuft, und wenn sich das hintere Mantelteil (32) in der hinteren Position befindet, sich die Nadel/Kanüle (5) in einer Beseitigungsposition befindet, wobei sich das Vordersegment (51) in die Passage (21) zurückzieht, wobei das hintere Mantelteil (32), das die Achse umgibt und eine Rücklaufkammer (Flashback-Kammer) (36) definiert, die mit der Nadel/Kanüle (5) in flüssiger Kommunikation steht,
ein freigebbares Sicherungselement, das zum Verhindern der axialen Bewegung des Nadelansatzes (3) relativ zum Zylinder (2) angeordnet ist, wenn sich das hintere Mantelteil (32) in der vorderen Position befindet, und welches Folgendes einschließt:
ein Sicherungsloch (231), welches in der äußeren Zylinderwandoberfläche (222) des Wandteils (229) mit größerem Durchmesser gebildet ist, und in einer radialen Richtung durch die innere Zylinderwandoberfläche (221) verläuft, und
einen Einraststift (33), der so angeordnet ist, dass er in der radialen Richtung verläuft, und in das Sicherungsloch (231) einrasten kann, um ein miteinander Einrasten zwischen dem Wandteil (229) mit größerem Durchmesser und dem hinteren Mantelteil (32) dergestalt zu etablieren, dass eine Bewegung des hinteren Mantelteils (32) an der vorderen Position verhindert wird;
ein Betätigungselement (34), das extern betätigt werden kann und angeordnet ist, um zu ermöglichen, dass der Einraststift (33) aus dem Sicherungsloch (231) dergestalt gelöst werden kann, um die axiale Bewegung des Nadelansatzes (3) in die hintere Position zu erlauben;
einen Katheteransatz (41), der ein Hülsenteil (411) einschließt, das abnehmbar auf das Wandteil (228) mit kleinerem Durchmesser [relativ zum vorderen Halteteil (31) des Nadelansatzes (3)] gestülpt ist und das einen Kanal (412) entlang der Achse definiert, und ein Spitzenteil (413), das sich gegenüber dem Hülsenteil (411) entlang der Achse befindet, und das ein durchgehendes Loch (414) definiert, das sich mit dem Kanal (412) entlang der Achse in Kommunikation befindet und das die Verlängerung des vorderen Segments (51) dort hindurch erlaubt; und
einen rohrförmigen Katheter (43) mit einem proximalen Segment (431), das in das durchgehende Loch (414) insertiert wird und das entlang der Achse verläuft, um in flüssige Kommunikation mit dem Kanal (412) zu treten, und einem distalen Segment (432), das von dem proximalen Segment (431) entlang der Achse verläuft, um vom Spitzenteil (413) dergestalt nach vom zu verlaufen, um das Vordersegment (51) der Nadel/Kanüle (5) zu umgeben und zu verkleiden, während dem Spitzenende (52) erlaubt wird, sich vom distalen Segment (432) nach vorn zu verlängern, wenn die Nadel/Kanüle (5) in die gebrauchsbereite Position gebracht wird, **dadurch gekennzeichnet, dass** das vordere Halteteil (31) vom Wandteil (228) mit kleinerem Durchmesser umgeben ist, wenn es sich in der vorderen Position befindet.

2. Intravenöse Kathetereinführvorrichtung nach Anspruch 1, **dadurch gekennzeichnet, dass** der Nadelansatz (3) weiter ein Zwischenteil (38) einschließt, welches das vordere Halteteil (31) und das hintere Mantelteil (32) miteinander verbindet, damit die Nadel/Kanüle (5) mit der Rücklaufkammer (36) in Kommunikation tritt und die lichtdurchlässig ist, um die Sicht des dort hindurch fließenden Blutes zu erlauben.

3. Intravenöse Kathetereinführvorrichtung nach Anspruch 2, **dadurch gekennzeichnet, dass** der Nadelansatz (3) weiter ein luftdurchlässiges Element (37) einschließt, welches sich mit dem hinteren Mantelteil (32) im Eingriff befindet, um auf diese Weise die Rücklaufkammer (36) zu schließen.

4. Intravenöse Kathetereinführvorrichtung nach Anspruch 3, **dadurch gekennzeichnet, dass** das luftdurchlässige Element (37) aus einem porösen Filtermaterial hergestellt ist.

5. Intravenöse Kathetereinführvorrichtung nach Anspruch 1, **dadurch gekennzeichnet, dass** das hintere Wandteil (229) mit größerem Durchmesser eine verlängerte Führungsbahn (23) aufweist, die von der äußeren Zylinderwandoberfläche (222) durch die innere Zylinderwandoberfläche (221) in der radialen Richtung und verlängernd vom Sicherungsloch (231) nach hinten verläuft und in der Längsrichtung an einem hinteren Sicherungsende (232) endet,
wobei der Einraststift (33) auf dem hinteren Mantelteil (32) angeordnet ist und radial von diesem verläuft, um an einem verschobenen Ende zu enden, das radial und nach außen von der äußeren Zylinderwandoberfläche (222) verläuft und der verlängerten Führungsbahn (23) vom Sicherungsloch (231) bis zum hinteren Sicherungsende (232) entlang gleitbar ist, wenn das hintere Mantelteil (32) des Nadelansatzes (3) von der vorderen Position zur hinteren Position gleitet,
wobei das Betätigungselement (34) an das verschobene Ende des Einraststifts (33) angeschlossen ist und von der äußeren Zylinderwandoberfläche (222) nach außen angeordnet und relativ zu ihr gleitbar ist.

6. Intravenöse Kathetereinführvorrichtung nach Anspruch 5, **dadurch gekennzeichnet, dass** die verlängerte Führungsbahn (23) vordere und hintere sich verjüngende Regionen (24, 25) aufweist, die unmittelbar hinter dem Sicherungsloch (231) bzw. unmittelbar vor dem hinteren Sicherungsende (232) dergestalt gebildet sind, dass der Einraststift (33), sobald er durch eine der vorderen und hinteren sich verjüngenden Regionen (24, 25) gepresst wurde, die Bewegung des Einraststifts (33) mittels einer Einschnappvorrichtung (Snap-fit) in einem entsprechenden einen von dem Sicherungsloch (231) und dem hinteren Sicherungsende (232) verhindert wird, um den Nadelansatz (3) in eine entsprechende von einer der vorderen und hinteren Positionen zu bringen.

7. Intravenöse Kathetereinführvorrichtung nach Anspruch 6, **dadurch gekennzeichnet, dass** das hintere Wandteil (229) mit größerem Durchmesser ferner einen Schlitz (27) aufweist, der vom hinteren Sicherungsende (232) der verlängerten Führungsbahn (23) zum hinteren offenen Ende (223) verläuft.

8. Intravenöse Kathetereinführvorrichtung nach Anspruch 5, **dadurch gekennzeichnet, dass** das Sicherungsloch (231) ein proximales Verbindungsende (231a) und ein distales Sicherungsende (231b) einschließt, die sich miteinander in einer Querrichtung relativ zur Längsrichtung gegenüberliegen und die sich proximal zur bzw. distal von der verlängerten Führungsbahn (23) dergestalt befinden, dass der Einraststift (33) im distalen Sicherungsende (231b) eingerastet ist, um die Bewegung des hinteren Mantelteils (32) des Nadelansatzes (3) in der vorderen Position und dergestalt zu verhindern, dass das Betätigungselement (34) betätigt wird, um den Einraststift (33) aus dem distalen Sicherungsende (231b) zum proximalen Verbindungsende (231a) dergestalt zu bewegen, um eine gleitbare Bewegung des Einraststifts (33) entlang der verlängerten Führungsbahn (23) zu erlauben.

9. Intravenöse Kathetereinführvorrichtung nach Anspruch 8, weiter **gekennzeichnet durch** ein Diskriminationselement (7), das zwischen das hintere Mantelteil (32) und die innere Zylinderwandoberfläche (221) positioniert ist, und das zum Diskriminieren des Nadelansatzes (3) in Richtung der hinteren Position angeordnet ist.

10. Intravenöse Kathetereinführvorrichtung nach Anspruch 9, **dadurch gekennzeichnet, dass** die innere Zylinderwandoberfläche (221) des Wandteils (229) mit größerem Durchmesser bzw. dem hinteren Mantelteil (32) einen ringförmigen Bund (227) und einen Flansch (323) aufweisen, die sich gegenüber voneinander befinden und in der Längsrichtung einander zugekehrt sind, um einen Raum zum Aufnehmen eines Diskriminationselements dort dazwischen zu definieren, wobei das Diskriminationselement (7) eine gewundene Feder (7) darstellt, die vordere und hintere Federenden aufweist, die gegen den ringförmigen Bund (227) bzw. den Flansch (323) dergestalt stoßen, dass die gewundene Feder (7) durch den Nadelansatz (3) komprimiert wird, wenn sich das hintere Mantelteil (32) in der vorderen Position befindet.

11. Intravenöse Kathetereinführvorrichtung nach Anspruch 9, **dadurch gekennzeichnet, dass** das hintere Mantelteil (32) bzw. die innere Zylinderwandoberfläche (221) des Wandteils (229) mit größerem Durchmesser einen ringförmigen Flansch (39) und eine Kante (220) aufweisen, die sich gegenüberliegen und die in der Längsrichtung dergestalt einander zugekehrt sind, um einen Raum zum Aufnehmen des Diskriminationselements (7) dort dazwischen zu definieren, wobei das Diskriminationselement (7) eine gewundene Feder (7) darstellt, die vordere und hintere Federenden aufweist, die an dem ringförmigen Flansch (39) bzw. der Kante (220) dergestalt sicher befestigt sind, dass die gewundene Feder (7) durch den Nadelansatz (3) gespannt wird, wenn sich das hintere Mantelteil (32) in der vorderen Position befindet.

12. Intravenöse Kathetereinführvorrichtung nach Anspruch 1, **dadurch gekennzeichnet, dass** das Betätigungselement (34) ein Triggerelement (6) einschließt, das an der äußeren Zylinderwandoberfläche (222) an einem Drehpunkt (63) schwenkbar befestigt ist und das ein gewichtetes Ende (61) einschließt, das integral mit dem Einraststift (33) gebildet ist und das vom hinteren Mantelteil (32) dergestalt nach hinten angeordnet ist, damit der Einraststift (33) gegen das hintere Mantelteil (32) anstößt, wenn sich die Nadel/Kanüle (5) in der gebrauchsbereiten Position befindet, und ein Betätigungsende (62), das an der gegenüberliegenden Seite des gewichteten Endes (61) relativ zum Drehpunkt (63) positioniert ist, um auf diese Weise zum Bewegen des Einraststifts (33) in der radialen Richtung betätigt zu werden, um den Einraststift (33) aus der Passage (21) zurückzuziehen; und
ein Diskriminationselement (7), das zwischen dem hinteren Mantelteil (32) und der inneren Zylinderwandoberfläche (221) zum Diskriminieren des Nadelansatzes (3) in Richtung der rückseitigen Position angeordnet ist.

## Revendications

1. Dispositif d'insertion d'un cathéter intraveineux comprenant :
un barillet (2) ayant des extrémités avant et arrière ouvertes (224, 223) situées à l'opposé l'une de l'autre dans une direction longitudinale et une paroi entourant le barillet (22) qui est disposée entre lesdites extrémités avant et arrière ouvertes (224, 223) et les interconnecte, ladite paroi entourant le barillet (22) comportant une portion avant de paroi de plus faible diamètre (228) et une portion arrière de paroi de plus gros diamètre (229) qui sont à l'opposé l'une de l'autre en direction longitudinale et qui sont à la proximité desdites extrémités avant et arrière ouvertes (224, 223), respectivement, ladite paroi entourant le barillet (22) ayant une surface interne de paroi du barillet (221) qui entoure un axe en direction longitudinale et renferme un passage (21) qui est en communication avec lesdites extrémités avant et arrière ouvertes (224, 223), et une surface externe de paroi du barillet (222) à l'opposé de ladite surface interne de paroi du barillet (221) dans des directions radiales par rapport à l'axe ;
une canule à aiguille (5) ayant un segment avant (51) qui se termine à une extrémité de tête (52) et présente une extrémité de connexion arrière (53) à l'opposé dudit segment avant (51) le long de l'axe ;
une garde d'aiguille (3) comportant une portion de retenue avant (31) et une portion d'enveloppe arrière (32) disposées à l'opposé l'une de l'autre le long de l'axe, ladite portion de retenue avant (31) étant reçue dans ledit passage (21) entouré par ladite portion de paroi de plus faible diamètre (228), ladite portion d'enveloppe arrière (32) étant insérée dans ledit passage (21) à partir de ladite extrémité arrière ouverte (223) et pouvant être coulissée par rapport à ladite paroi entourant le barillet (22) le long de l'axe entre des positions avant et arrière de façon à se trouver à proximité de ladite extrémité avant ouverte (224) et de ladite extrémité arrière ouverte (223), respectivement, ladite portion de retenue avant (31) maintenant ladite extrémité de connexion arrière (53) de ladite canule à aiguille (5) de façon telle que quand ladite portion d'enveloppe arrière (32) est en position avant, ladite canule à aiguille (5) est placée dans une position d'utilisation où ledit segment avant (51) se projette en avant de ladite extrémité avant ouverte (224) prête à l'emploi, et de façon telle que quand ladite portion d'enveloppe arrière (32) est en position arrière, ladite canule à aiguille (5) est placée dans une position d'élimination où ledit segment avant (51) est ramené dans ledit passage (21), ladite portion d'enveloppe arrière (32) entourant l'axe et définissant une chambre de retour (36) qui est en communication liquidienne avec ladite canule à aiguille (5) ;
un élément de retenue libérable qui est disposé de façon à stopper le déplacement axial de ladite garde d'aiguille (3) par rapport au dit barillet (2) quand ladite portion d'enveloppe arrière (32) est en position avant, et qui comprend
un orifice de retenue (231) ménagé dans ladite surface externe de paroi du barillet (222) de ladite portion de paroi de plus gros diamètre (229) et se projetant en direction radiale au travers de ladite surface interne de paroi du barillet (221) ; et
une clavette d'enclenchement (33) qui est disposée de façon à se projeter en direction radiale et qui peut être enclenchée dans ledit orifice de retenue (231) pour établir un enclenchement réciproque entre ladite portion de paroi de plus gros diamètre (229) et ladite portion d'enveloppe arrière (32) de façon telle que le déplacement de ladite portion d'enveloppe arrière (32) en position avant est stoppé ;
une commande (34) actionnable de l'extérieur et disposée de façon à permettre la déconnexion de la clavette d'enclenchement (33) dudit orifice de retenue (231) pour rendre possible le déplacement axial de ladite garde d'aiguille (3) vers la position arrière ;
un pavillon du cathéter (41) comprenant une portion manchon (411) qui s'emboîte de façon détachable sur ladite portion de paroi de plus faible diamètre (228) [par rapport à ladite portion de retenue avant (31) de ladite garde d'aiguille (3)] et qui définit un conduit (412) le long de l'axe, et une portion de tête (413) qui est à l'opposé de ladite portion manchon (411) le long de l'axe et définit un orifice débouchant (414) qui communique avec ledit conduit (412) le long de l'axe et au travers duquel une extension dudit segment avant (51) est possible ; et
un cathéter tubulaire (43) ayant un segment proximal (431) qui est inséré dans ledit orifice débouchant (414) et qui se prolonge le long de l'axe pour être en communication liquidienne avec ledit conduit (412), et un segment distal (432) qui se prolonge à partir dudit segment proximal (431) le long de l'axe et se projette en avant de ladite portion de tête (413) de façon à entourer et envelopper ledit segment avant (51) de ladite canule à aiguille (5) tout en permettant à ladite extrémité de tête (52) de se projeter en avant dudit segment distal (432) quand ladite canule à aiguille (5) est placée dans la position d'utilisation, **caractérisé en ce que** ladite portion de retenue avant (31) est entourée par ladite portion de paroi de plus faible diamètre (228) quand elle est en position avant.

2. Dispositif d'insertion d'un cathéter intraveineux de la revendication 1, **caractérisé en ce que** ladite garde d'aiguille (3) comprend également une portion intermédiaire (38) qui interconnecte ladite portion de retenue avant (31) et ladite portion d'enveloppe arrière (32) pour faire communiquer ladite canule à aiguille (5) avec ladite chambre de retour (36) et qui est translucide à la lumière et permet la visualisation du flux de sang.

3. Dispositif d'insertion d'un cathéter intraveineux de la revendication 2, **caractérisé en ce que** ladite garde d'aiguille (3) comprend également un élément perméable à l'air (37) qui est en enclenchement avec ladite portion d'enveloppe arrière (32) de façon à fermer ladite chambre de retour (36).

4. Dispositif d'insertion d'un cathéter intraveineux de la revendication 3, **caractérisé en ce que** ledit élément perméable à l'air (37) est composé d'un matériau filtrant poreux.

5. Dispositif d'insertion d'un cathéter intraveineux de la revendication 1, **caractérisé en ce que** ladite portion arrière de paroi de plus gros diamètre (229) présente une glissière allongée (23) qui s'étend à partir de la surface externe de paroi du barillet (222) au travers de ladite surface interne de paroi du barillet (221) en direction radiale, et se prolonge à partir dudit orifice de retenue (231) vers l'arrière et en direction longitudinale pour se terminer à une extrémité de retenue arrière (232),
ladite clavette d'enclenchement (33) étant disposée sur et se projetant radialement à partir de ladite portion d'enveloppe arrière (32) pour se terminer à une extrémité mobile qui se projette radialement et vers l'extérieur de ladite surface externe de paroi du barillet (222) et pouvant être coulissée le long de ladite glissière allongée (23) dudit orifice de retenue (231) à ladite extrémité de retenue arrière (232) quand ladite portion d'enveloppe arrière (32) de ladite garde d'aiguille (3) coulisse de la position avant à la position arrière ;
ladite commande (34) étant connectée à ladite extrémité mobile de ladite clavette d'enclenchement (33) et étant disposée vers l'extérieur de ladite surface externe de paroi du barillet (222) et pouvant être coulissée par rapport à celle-ci.

6. Dispositif d'insertion d'un cathéter intraveineux de la revendication 5, **caractérisé en ce que** ladite glissière allongée (23) présente des régions avant et arrière resserrées (24, 25) qui sont ménagées immédiatement en aval dudit orifice de retenue (231) et immédiatement en amont de ladite extrémité de retenue arrière (232), respectivement, de façon telle qu'une fois que ladite clavette d'enclenchement (33) est forcée au travers de l'une desdites régions avant et arrière resserrées (24, 25), le déplacement de ladite clavette d'enclenchement (33) est stoppé par emboîtement élastique dans ledit orifice de retenue (231) ou dans ladite extrémité de retenue arrière (232) correspondant de façon à placer la garde d'aiguille (3) dans la position avant ou arrière correspondante.

7. Dispositif d'insertion d'un cathéter intraveineux de la revendication 6, **caractérisé en ce que** ladite portion arrière de paroi de plus gros diamètre (229) présente également une fente (27) qui s'étend de ladite extrémité de retenue arrière (232) de ladite glissière allongée (23) à ladite extrémité de retenue arrière (223).

8. Dispositif d'insertion d'un cathéter intraveineux de la revendication 5, **caractérisé en ce que** ledit orifice de retenue (231) comprend une extrémité de connexion proximale (231a) et une extrémité de retenue distale (231b) situées à l'opposé l'une de l'autre en direction transversale par rapport à la direction longitudinale et qui sont respectivement proximale et distale à ladite glissière allongée (23) de façon telle que ladite clavette d'enclenchement (33) est enclenchée dans ladite extrémité de retenue distale (231b) pour stopper le déplacement de ladite portion d'enveloppe arrière (32) de ladite garde d'aiguille (3) en position avant, et de façon telle que ladite commande (34) est actionnée pour déplacer ladite clavette d'enclenchement (33) de ladite extrémité de retenue distale (231b) à ladite extrémité de connexion proximale (231a) pour permettre le coulissement de ladite clavette d'enclenchement (33) le long de ladite glissière allongée (23).

9. Dispositif d'insertion d'un cathéter intraveineux de la revendication 8, également **caractérisé en ce qu'**il présente un élément de sollicitation (7) qui est interposé entre ladite portion d'enveloppe arrière (32) et ladite surface interne de paroi du barillet (221) et qui est disposé de façon à solliciter le déplacement de ladite garde d'aiguille (3) vers la position arrière.

10. Dispositif d'insertion d'un cathéter intraveineux de la revendication 9, **caractérisé en ce que** ladite surface interne de paroi du barillet (221) de la portion de paroi de plus gros diamètre (229) et ladite portion d'enveloppe arrière (32) comportent, respectivement, un épaulement annulaire (227) et une collerette (323) qui sont situés à l'opposé et en confrontation l'un par rapport à l'autre en direction longitudinale de façon à définir entre eux un espace abritant un élément de sollicitation, ledit élément de sollicitation (7) correspondant à un ressort à boudin (7) dont les extrémités avant et arrière butent contre ledit épaulement annulaire (227) et ladite collerette (323), respectivement, de sorte que le ressort à boudin (7) est comprimé par ladite garde d'aiguille (3) quand ladite portion d'enveloppe arrière (32) est en position avant.

11. Dispositif d'insertion d'un cathéter intraveineux de la revendication 9, **caractérisé en ce que** ladite portion d'enveloppe arrière (32) et ladite surface interne de paroi du barillet (221) de la portion de paroi de plus gros diamètre (229) comportent, respectivement, une collerette annulaire (39) et une arête (220) qui sont situées à l'opposé et en confrontation l'une par rapport à l'autre en direction longitudinale de façon à définir entre elles un espace abritant un élément de sollicitation, ledit élément de sollicitation (7) correspondant à un ressort à boudin (7) dont les extrémités avant et arrière sont ancrées à ladite collerette annulaire (39) et à ladite arête (220), respectivement, de sorte que le ressort à boudin (7) est tendu par ladite garde d'aiguille (3) quand ladite portion d'enveloppe arrière (32) est en position avant.

12. Dispositif d'insertion d'un cathéter intraveineux de la revendication 1, **caractérisé en ce que** ladite commande (34) comprend un élément de déclenchement (6) qui est monté de manière pivotante sur ladite surface externe de paroi du barillet (222) en un point d'appui (63) et comprend une extrémité à poids (61) qui est formée intégralement avec ladite clavette d'enclenchement (33) et qui est disposée à l'arrière de ladite portion d'enveloppe arrière (32) de façon à faire buter ladite clavette d'enclenchement (33) contre ladite portion d'enveloppe arrière (32) quand ladite canule à aiguille (5) est en position d'utilisation, et une extrémité motrice (62) disposée sur un côté opposé de ladite extrémité à poids (61) par rapport au dit point d'appui (63) de telle sorte que son actionnement produit le déplacement de ladite clavette d'enclenchement (33) en direction radiale et son retrait dudit passage (21) ;
un élément de sollicitation (7) qui est disposé entre ladite portion d'enveloppe arrière (32) et ladite surface interne de paroi du barillet (221) de façon à solliciter le déplacement de ladite garde d'aiguille (3) vers la position arrière.
